(19) Europäisches Patentamt
European Patent Office
Office européen des brevets

(11) EP 1 144 356 B1

(12) FASCICULE DE BREVET EUROPEEN

(45) Date de publication et mention de la délivrance du brevet: **20.08.2003 Bulletin 2003/34**

(21) Numéro de dépôt: **00900624.8**

(22) Date de dépôt: **20.01.2000**

(51) Int Cl.⁷: **C07C 213/08**, C07C 219/08

(86) Numéro de dépôt international: **PCT/FR00/00122**

(87) Numéro de publication internationale: **WO 00/043346 (27.07.2000 Gazette 2000/30)**

(54) **PROCEDE DE FABRICATION DE SOLUTIONS AQUEUSES DE SELS INSATURES D'AMMONIUM QUATERNAIRE**

VERFAHREN ZUR HERSTELLUNG VON WÄSSRIGEN LÖSUNGEN VON UNGESÄTTIGTEN QUATERNÄREN AMMONIUMSALZEN

METHOD FOR MAKING AQUEOUS SOLUTIONS OF UNSATURATED QUATERNARY AMMONIUM SALTS

(84) Etats contractants désignés:
**AT BE CH CY DE DK ES FI FR GB GR IE IT LI LU MC NL PT SE**

(30) Priorité: **21.01.1999 FR 9900641**

(43) Date de publication de la demande: **17.10.2001 Bulletin 2001/42**

(73) Titulaire: **Atofina**
**92800 Puteaux (FR)**

(72) Inventeurs:
- **RIONDEL, Alain**
  **F-57600 Forbach (FR)**
- **HERBST, Gilles**
  **F-57350 Spicheren (FR)**
- **ESCH, Marc**
  **F-57800 Freyming-Merlebach (FR)**
- **DELAUNAY, Eric**
  **F-31320 Rebigue (FR)**
- **MEYER, Peter**
  **F-75020 Paris (FR)**

(74) Mandataire: **Rieux, Michel**
**Atofina,**
**(D.R.D.C./D.P.I.),**
**4/8 cours Michelet**
**92800 Puteaux (FR)**

(56) Documents cités:
**EP-A- 0 250 325**
**EP-A- 0 329 512**
**EP-A- 0 818 437**
**EP-A- 0 819 671**
**WO-A-89/07588**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention porte sur la fabrication de solutions aqueuses de sels insaturés d'ammonium quaternaire (ci-après dénommés sels quaternaires) , répondant à la formule (I) suivante :

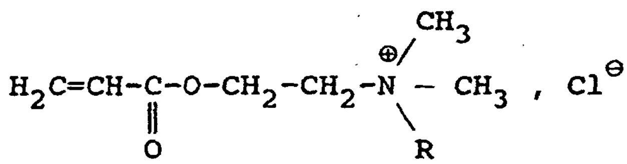

(I)

dans laquelle R représente un radical méthyle ou benzyle, par réaction, en présence d'eau, de l'acrylate de N,N-diméthylaminoéthyle (ADAME) avec un agent quaternisant de formule (II) :

R - Cl (II)

dans laquelle R est tel que défini ci-dessus.

On utilise des solutions aqueuses de sels quaternaires (I) pour préparer des polymères destinés à servir de floculants cationiques dans le traitement des eaux.

Le brevet européen EP-B-250 325 décrit un procédé de préparation de solutions aqueuse de sels quaternaires dont ceux de formule (I), procédé selon lequel, en présence d'au moins un inhibiteur de polymérisation :

- dans une première étape (a), on fait réagir l'ADAME avec 5 à 20% en poids de la quantité pondérale nécessaire à la réaction de l'agent quaternisant, ou, suivant une variante (a'), avec 5 à 20% en poids, par rapport au poids de l'ADAME, d'une solution aqueuse de sels quaternaires, laquelle comprend de 50 à 85% en poids de sels quaternaires ; et
- dans une deuxième étape (b), on ajoute en continu l'eau et l'agent quaternisant jusqu'à l'obtention de la concentration souhaitée de sels quaternaires dans l'eau.

Pendant les étapes (a) et (b), on maintient la température à une valeur comprise entre 30 et 60°C. De plus, pendant les étapes (a) et (b) et, en particulier, à l'approche de la fin de la réaction, on maintient dans le milieu réactionnel un courant de gaz oxygéné tel que le rapport en volume (ou débit volumétrique) de gaz total à la sortie du réacteur sur le volume (ou débit volumétrique) d'oxygène introduit à l'entrée de ce même réacteur est inférieur à 100.

Ce procédé permet de préparer des solutions aqueuses de sels quaternaires qui ont une stabilité à température ambiante supérieure à un an. Toutefois, on constate, dans ces solutions, une teneur particulièrement élevée d'impuretés, en particulier de

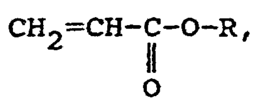

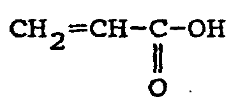

et d'ADAME. En outre, ce procédé nécessite des temps de réaction relativement longs, ce qui représente un inconvénient économique évident.

Dans la demande internationale WO 89/07 588, a alors été proposé un procédé destiné à réduire la formation des impuretés lors de la réaction de quaternisation. Conformément à ce procédé, la réaction est effectuée à une température comprise entre 10 et 80°C, et

(a) dans une première étape, on introduit dans le réacteur la totalité ou une partie de l'agent quaternisant nécessaire à la réaction, cet agent étant à l'état liquide dans les conditions de la réaction,

(b) ensuite, on ajoute l'ADAME, et

(c) dès que 0 à 30% de la stoechiométrie de l'ADAME ont été introduits dans le réacteur, on ajoute en continu et simultanément le reste d'agent quaternisant, le reste d'ADAME et l'eau jusqu'à l'obtention de la concentration souhaitée de sels quaternaires,

(d) et, dans le cas où l'agent quaternisant est introduit à l'état gazeux à la température de réaction, la réaction est effectuée en présence d'oxygène et on impose une pression de manière que l'agent quaternisant soit liquide à la température de réaction, et, en fin de réaction, on diminue progressivement la pression jusqu'à la pression atmosphérique et simultanément on impose un rapport en débit volumétrique de gaz total à la sortie du réacteur sur le débit volumétrique d'oxygène introduit dans le réacteur inférieur à 100.

Le procédé ci-dessus selon WO 89/07 588 apporte des améliorations notables au procédé selon EP-B-250 325. Cependant, il est apparu que la pureté avec laquelle on obtient les sels quaternaires est encore insuffisante. Ainsi; au cours de la réaction de l'ADAME avec $CH_3Cl$ en milieu aqueux, conduisant au sel désigné également dans ce qui suit par l'abréviation ADAMQUAT MC, il se forme, comme impuretés, outre l'acide acrylique (AA) formé par hydrolyse de l'ADAME, le dimère de l'ADAMQUAT MC, représenté par la formule (1) :

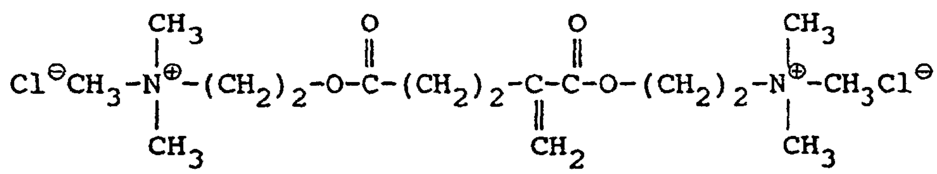 (1).

Grâce à une série de tests de réactivité en polymérisation, il a pu être démontré que ces impuretés affectaient la qualité des polymères cationiques dérivés de l'ADAMQUAT.

La Société déposante a donc recherché des conditions opératoires de préparation de solutions aqueuses du sel de formule (I), qui soient capables de minimiser les impuretés précitées, de façon à proposer un sel (I) en solution aqueuse de très haute qualité analytique.

Ce nouveau procédé, qui fait donc l'objet de la présente invention, est caractérisé par le fait que :

(a) dans un réacteur fermé qui contient la totalité de l'ADAME et qui a été pressurisé par de l'air ou de l'air appauvri à 0,5 à 3 bars, on conduit la réaction en introduisant en continu, à la température de. 35 à 65°C, en particulier de 40 à 60°C, d'une part, l'agent quaternisant (II) et, d'autre part, l'eau, jusqu'à l'obtention de la concentration souhaitée en sel (I) dans l'eau, le démarrage de l'introduction de l'eau commençant lorsque l'on a ajouté 0 - 20%, en particulier 5 - 15% de la quantité pondérale nécessaire à la réaction de l'agent quaternisant (II), et la pression en fin de réaction pouvant atteindre 9 bars, en particulier 4 à 7 bars ; puis

(b) on dépressurise le réacteur tout en maintenant une teneur en oxygène constante par introduction simultanée d'air, et après retour à la pression atmosphérique, on élimine l'agent quaternisant résiduaire, par exemple par strippage à l'air.

Conformément à d'autres caractéristiques particulières du procédé selon l'invention :

- on introduit l'agent quaternisant pendant un laps de temps de 1 - 7 heures et l'eau pendant un laps de temps de 2 - 8 heures ;
- on conduit la réaction avec un rapport molaire de l'agent quaternisant à l'ADAME de 1 à 1,1, de préférence de 1 à 1,05 ;
- on conduit la réaction avec un rapport moyen de débit eau/agent quaternisant de 0,1 - 1,2 en particulier de 0,3 - 0,8.

Le procédé selon l'invention permet notamment de préparer des solutions aqueuses ayant une concentration en sels quaternaires (I) de 50 à 85% en poids, et contenant des quantités très faibles d'impuretés, comme illustré dans le Tableau 1 ci-après.

Par ailleurs, le procédé selon la présente invention peut être conduit en présence d'au moins un stabilisant, lequel peut être choisi parmi le 3,5-ditert.-butyl-4-hydroxytoluène, l'éther méthylique d'hydroquinone, l'hydroquinone, le catéchol, le tert.-butyl catéchol, la phénothiazine, et les mélanges de ces stabilisants, la teneur en agent(s) stabilisant (s) étant notamment de 20 à 2000 ppm, de préférence de 100 à 1200 ppm, par rapport à la solution aqueuse de sel quaternaire (I).

On peut ajouter en outre au milieu réactionnel au moins un agent séquestrant pour métaux, choisi notamment

parmi l'acide diéthylène triamine penta acétique, le sel pentasodique de l'acide diéthylène triamine penta acétique, l'acide N-hydroxyéthyl-éthylène diamine triacétique et le sel trisodique de l'acide N-hydroxyéthyl-éthylène diamine triacétique, la teneur en agent(s) séquestrant(s) étant notamment de 1 à 100 ppm, de préférence de 5 à 30 ppm, par rapport à la solution aqueuse de sel quaternaire (I).

D'une manière générale, les agents séquestrants sont ajoutés sous la forme d'une solution aqueuse, car ils sont généralement disponibles sous cette forme. Ainsi, le sel pentasodique de l'acide diéthylène triamine penta acétique commercialisé sous la dénomination VERSENEX 80 se présente sous la forme d'une solution aqueuse à 40% en poids environ.

Les Exemples suivants illustrent la présente invention sans toutefois en limiter la portée. Des ces exemples, les pourcentages sont en poids sauf indication contraire.

EXEMPLE 1

Dans un réacteur en verre de 1 l, à double enveloppe, spécialement conçu pour tenir la pression, équipé d'une sonde de température, d'un agitateur spécifique gaz liquide (turbine avec axe creux), d'une soupape tarée à 10 bars, d'un disque d'explosion et de cannes plongeantes pour l'introduction des différents réactifs, on a chargé 429 g d'ADAME. Le réacteur a été fermé, puis pressurisé avec 1 bar d'air appauvri. L'agitation et le chauffage ont été mis en service.

Dès que la température a atteint 40°C (température de consigne : 47°C), on a commencé à introduire $CH_3Cl$ à raison de 159 g/h. Dès que l'on a ajouté 15 g de $CH_3Cl$, soit 10% de la stoechiométrie en $CH_3Cl$, on a démarré l'introduction d'eau à un débit de 60 g/h tout en maintenant l'ajout de $CH_3Cl$. Le rapport de débit $H_2O/CH_3Cl$ a été maintenu constant à 0,37 tout au long de la réaction. Lorsque toute l'eau a été introduite (soit 143 g), le réacteur a été ramené à la pression atmosphérique en utilisant le protocole suivant :

- dégazage du $CH_3Cl$ en excès pendant 30 minutes avec introduction simultanée d'air dans la charge (débit : 3 Nl/h) ;
- retour progressif à la pression atmosphérique.

Les traces de $CH_3Cl$ ont ensuite été éliminées par stripping à l'air (débit : 5Nl/h) pendant 30 minutes. Le réacteur a ensuite été mis en refroidissement, puis vidangé.

Le brut réactionnel (716 g) a été analysé par chromatographie liquide haute performance (HPLC) pour déterminer les teneurs en acide acrylique et en composé (1). Les résultats sont présentés dans le Tableau 1.

Les durées des différentes phases de la réaction étaient les suivantes :

- introduction $CH_3Cl$ : 1 h
- introduction $H_2O$ : 2,3 h
- dégazage : 0,5 h
- stripping : 0,5 h

soit une durée totale d'environ 3,5 h

EXEMPLES 2 à 4

On a procédé comme à l'Exemple 1, excepté que l'on a fait varier le débit de $CH_3Cl$.

Les résultats sont présentés dans le Tableau 1.

EXEMPLES 5 à 7

On a procédé comme à l'Exemple 3, excepté que l'on a commencé à introduire l'eau dès le début.

EXEMPLES 8 à 10

On a procédé comme à l'Exemple 3, excepté que l'on a fait varier la température.

Les résultats sont présentés dans le Tableau 1.

TABLEAU 1

$[CH_3Cl]/[ADAME]$ = 1,05 ; Pression maximale : 6 bars ; ADAME : 429 g (3 moles) ; $CH_3Cl$ = 159,1 g (3,15 moles)

| Exemple | Masse du brut réactionnel (g) | Durée introduction $CH_3Cl$ (h) | T (°C) | Rapport moyen de débit $H_2O/CH_3Cl$ | Analyse HPLC (ppm) | | |
|---|---|---|---|---|---|---|---|
| | | | | | AA** | Dimère de l'ADAMQUAT MC de formule (1) *** | ADAMQUAT MC (%) **** |
| 1 | 716 | 1 | 47 | 0,37 | 267 | 125 | 81,8 |
| 2 | 716,5 | 2,25 | 47 | 0,7 | 810 | 397 | 80 |
| 3 | 699 | 4 | 47 | 0,85 | 990 | 1070 | 81,1 |
| 4 | 706 | 7,25 | 47 | 0,66 | 892 | 2145 | 80,2 |
| 5* | 717,5 | 4 | 47 | 0,5 puis 0,7 à mi-réaction | 769 | 727 | |
| 6* | 723,5 | 4 | 47 | 0,55 | 559 | 649 | |
| 7* | 691,5 | 4 | 47 | 0,38 puis 0,55 à mi-réaction | 561 | 698 | |
| 8 | 680 | 4 | 30 | 0,76 | 6259 | 12004 | 75 |
| 9 | 685,5 | 4 | 40 | 0,64 | 597 | 410 | 81,2 |
| 10 | 708,5 | 4 | 58 | 0,6 | 540 | 1360 | 78,9 |

* introduction de l'eau dès le début de la réaction ** AA = acide acrylique *** Teneur en dimère de l'ADAMQUAT MC de formule (1) exprimée arbitrairement en AA **** ADAMQUAT MC = solution aqueuse de chlorure d'acryloyloxyéthyltriméthylammonium.

## Revendications

1. Procédé de fabrication de solutions aqueuses de sels insaturés d'ammonium quaternaire répondant à la formule (I) suivante :

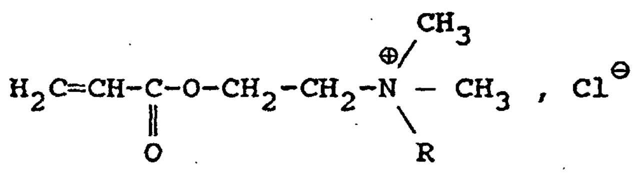

(I)

dans laquelle R représente un radical méthyle ou benzyle, par réaction, en présence d'eau, de l'acrylate de N,N-diméthylaminoéthyle (ADAME) avec un agent quaternisant de formule (II) :

R - Cl (II)

dans laquelle R est tel que défini ci-dessus,
**caractérisé par le fait que** :

(a) dans un réacteur fermé qui contient la totalité de l'ADAME et qui a été pressurisé par de l'air ou de l'air appauvri à 0,5 à 3 bars, on conduit la réaction en introduisant en continu, à la température de 35 à 65°C, d'une part l'agent quaternisant (II) et, d'autre part, l'eau, jusqu'à l'obtention de la concentration souhaitée en sel (I) dans l'eau, le démarrage de l'introduction de l'eau commençant lorsque l'on a ajouté 0 - 20% de la quantité pondérale nécessaire à la réaction de l'agent quaternisant (II), et la pression en fin de réaction pouvant atteindre 9 bars ; puis
(b) on dépressurise le réacteur tout en maintenant une teneur en oxygène constante par introduction simultanée d'air, et après retour à la pression atmosphérique, on élimine l'agent quaternisant résiduaire.

2. Procédé selon la revendication 1, **caractérisé par le fait qu'**on conduit la réaction à une température de 40 à 60°C.

3. Procédé selon l'une des revendications 1 et 2, **caractérisé par le fait qu'**on conduit la réaction avec une pression qui, en fin de réaction, atteint 4 à 7 bars.

4. Procédé selon l'une des revendications 1 à 3, **caractérisé par le fait qu'**on démarre l'introduction de l'eau lorsque l'on a ajouté 5 - 15% de la quantité pondérale nécessaire à la réaction de l'agent quaternisant (II).

5. Procédé selon l'une des revendications 1 à 3, **caractérisé par le fait que** l'on introduit l'agent quaternisant pendant un laps de temps de 1 - 7 heures et l'eau pendant un laps de temps de 2 - 8 heures.

6. Procédé selon l'une des revendications 1 à 5, **caractérisé par le fait que** l'on conduit la réaction avec un rapport molaire de l'agent quaternisant à l'ADAME de 1 à 1,1, de préférence de 1 à 1,05.

7. Procédé selon l'une des revendications 1 à 6, **caractérisé par le fait que** l'on conduit la réaction avec un rapport moyen de débit eau/agent quaternisant de 0,1 - 1,2, en particulier de 0,3 - 0,8.

8. Procédé selon l'une des revendications 1 à 7, **caractérisé par le fait qu'**il conduit à une solution aqueuse ayant une concentration en sel quaternaire (I) de 50 à 85% en poids.

9. Procédé selon l'une des revendications 1 à 8, **caractérisé par le fait qu'**il est conduit en présence d'au moins un stabilisant, choisi notamment parmi le 3,5-ditert.-butyl-4-hydroxytoluène, l'éther méthylique d'hydroquinone, l'hydroquinone, le catéchol, le tert.-butyl catéchol, la phénothiazine, et les mélanges de ces stabilisants, la teneur en agent(s) stabilisant(s) étant notamment de 20 à 2000 ppm, de préférence de 100 à 1200 ppm par rapport à la solution aqueuse de sel quaternaire (I).

10. Procédé selon la revendication 9, **caractérisé par le fait qu'**il est conduit en outre en présence d'au moins un

agent séquestrant pour métaux, choisi notamment parmi l'acide diéthylène triamine penta acétique, le sel pentasodique de l'acide diéthylène triamine penta acétique, l'acide N-hydroxyéthyl-éthylène diamine triacétique et le sel trisodique de l'acide N-hydroxyéthyl-éthylène diamine triacétique, la teneur en agent(s) séquestrant(s) étant notamment de 1 à 100 ppm, de préférence de 5 à 30 ppm, par rapport à la solution aqueuse de sel quaternaire (I).

**11.** Procédé selon l'une des revendications 1 à 10, **caractérisé par le fait que** l'on élimine l'agent quaternisant résiduaire par strippage à l'air.

## Patentansprüche

**1.** Verfahren zur Herstellung von wässrigen Lösungen von ungesättigten quartären Ammoniumsalzen der folgenden Formel (I):

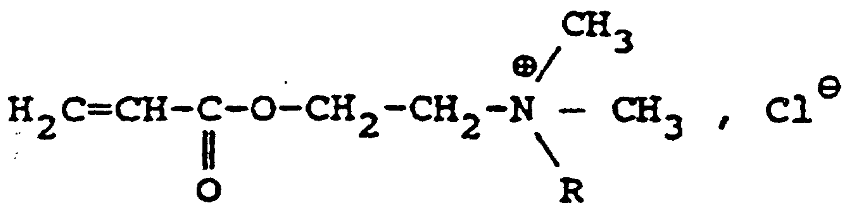

(I),

worin R Methyl oder Benzyl bedeutet, durch Umsetzung von N,N-Dimethylaminoethylacrylat (ADAME) mit einem Quaternisierungsmittel der folgenden Formel (II) in Gegenwart von Wasser:

$$R - Cl \quad (II),$$

worin R die oben angegebene Bedeutung aufweist, **dadurch gekennzeichnet, dass**:

(a) in einem geschlossenen Reaktor, der das gesamte ADAME enthält und der mit Luft oder abgereicherter Luft unter einen Druck von 0,5 bis 3 bar gesetzt ist, die Umsetzung durchgeführt wird, indem kontinuierlich bei einer Temperatur von 35 bis 65° C einerseits das Quaternisierungsmittel (II) und andererseits das Wasser zugeführt wird, bis die gewünschte Konzentration des Salzes (I) in Wasser erreicht ist, wobei mit der Wasserzugabe begonnen wird, wenn 0 bis 20% der für die Umsetzung erforderlichen Gewichtsmenge des Quaternisierungsmittels (II) zugegeben sind und wobei der Druck am Ende der Reaktion 9 bar erreichen kann; und dann
(b) der Druck in dem Reaktor gesenkt wird und dabei der Sauerstoffgehalt durch gleichzeitige Zufuhr von Luft konstant gehalten wird und nach dem Zurückkommen auf Atmosphärendruck das restliche Quaternisierungsmittel entfernt wird.

**2.** Verfahren nach Anspruch 1, **dadurch gekennzeichnet, dass** die Umsetzung bei einer Temperatur von 40 bis 60° C durchgeführt wird.

**3.** Verfahren nach einem der Ansprüche 1 und 2, **dadurch gekennzeichnet, dass** die Umsetzung bei einem Druck durchgeführt wird, der am Ende der Umsetzung 4 bis 7 bar erreicht.

**4.** Verfahren nach einem der Ansprüche 1 bis 3, **dadurch gekennzeichnet, dass** mit der Zugabe von Wasser begonnen wird, wenn 5 bis 15% der für die Umsetzung erforderlichen Gewichtsmenge des Quaternisierungsmittels (II) zugegeben sind.

**5.** Verfahren nach einem der Ansprüche 1 bis 3, **dadurch gekennzeichnet, dass** das Quaternisierungsmittel während einer Zeitspanne von 1 bis 7 Stunden und das Wasser während einer Zeitspanne von 2 bis 8 Stunden zugegeben wird.

6. Verfahren nach einem der Ansprüche 1 bis 5, **dadurch gekennzeichnet, dass** die Umsetzung mit einem Molverhältnis von Quaternisierungsmittel und ADAME von 1 bis 1,1 und vorzugsweise 1 bis 1,05 durchgeführt wird.

7. Verfahren nach einem der Ansprüche 1 bis 6, **dadurch gekennzeichnet, dass** die Umsetzung mit einem mittleren Durchsatzverhältnis Wasser/Quaternisierungsmittel von 0,1 bis 1,2 und insbesondere 0,3 bis 0,8 durchgeführt wird.

8. Verfahren nach einem der Ansprüche 1 bis 7, **dadurch gekennzeichnet, dass** es zu einer wässrigen Lösung mit einer Konzentration des quartären Salzes (I) von 50 bis 85 Gew.-% führt.

9. Verfahren nach einem der Ansprüche 1 bis 8, **dadurch gekennzeichnet, dass** es in Gegenwart mindestens eines Stabilisierungsmittels durchgeführt wird, das insbesondere unter 3,5-Di*t*-butyl-4-hydroxytoluol, Hydrochinonmethylether, Hydrochinon, Catechin, *t*-Butylcatechin, Phenothiazin und den Gemischen dieser Stabilisierungsmittel ausgewählt ist, wobei der Mengenanteil des Stabilisierungsmittels oder der Stabilisierungsmittel insbesondere im Bereich von 20 bis 2000 ppm und vorzugsweise 100 bis 1200 ppm, bezogen auf die wässrige Lösung des quartären Salzes (I), liegt.

10. Verfahren nach Anspruch 9, **dadurch gekennzeichnet, dass** es ferner in Gegenwart mindestens eines Maskierungsmittels für Metalle durchgeführt wird, das insbesondere unter Diethylentriaminpentaessigsäure, dem Pentanatriumsalz von Diethylentriaminpentaessigsäure, N-Hydroxyethyl-ethylendiamintriessigsäure und dem Trinatriumsalz von N-Hydroxyethylethylendiamintriessigsäure durchgeführt wird, wobei der Mengenanteil des Maskierungsmittels oder der Maskierungsmittel insbesondere im Bereich von 1 bis 100 ppm und vorzugsweise 5 bis 30 ppm, bezogen auf die wässrige Lösung des quartären Salzes (I), liegt.

11. Verfahren nach einem der Ansprüche 1 bis 10, **dadurch gekennzeichnet, dass** das restliche Quaternisierungsmittel durch Luft-Strippen entfernt wird.

## Claims

1. Process for the manufacture of aqueous solutions of unsaturated quaternary ammonium salts corresponding to the following formula (I):

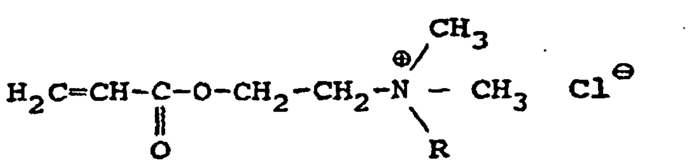

(I)

in which R represents a methyl or benzyl radical, by reaction, in the presence of water, of N,N-dimethylaminoethyl acrylate (DAMEA) with a quaternizing agent of formula (II):

$$R - Cl \qquad (II)$$

in which R is as defined above,
**characterized in that**:

(a) the reaction is carried out in a closed reactor, which comprises all of the DAMEA and which has been pressurized by air or depleted air to 0.5 to 3 bar, by continuously introducing, at a temperature of 35 to 65°C, on the one hand, the quaternizing agent (II) and, on the other hand, the water, until the desired concentration of salt (I) in the water is obtained, the start of the introduction of the water beginning when 0-20% of the amount by weight of the quaternizing agent (II) necessary for the reaction has been added, and it being possible for the pressure at the end of the reaction to reach 9 bar; then
(b) the reactor is depressurized while keeping a constant oxygen content by simultaneous introduction of air and, after returning to atmospheric pressure, the residual quaternizing agent is removed.

2. Process according to Claim 1, **characterized in that** the reaction is carried out at a temperature of 40 to 60°C.

3. Process according to either of Claims 1 and 2, **characterized in that** the reaction is carried out with a pressure which, at the end of the reaction, reaches 4 to 7 bar.

4. Process according to one of Claims 1 to 3, **characterized in that** the introduction of the water is started when 5-15% of the amount by weight of the quaternizing agent (II) necessary for the reaction has been added.

5. Process according to one of Claims 1 to 3, **characterized in that** the quaternizing agent is introduced over a period of time of 1-7 hours and the water is introduced over a period of time of 2-8 hours.

6. Process according to one of Claims 1 to 5, **characterized in that** the reaction is carried out with a molar ratio of the quaternizing agent to the DAMEA of 1 to 1.1, preferably of 1 to 1.05.

7. Process according to one of Claims 1 to 6, **characterized in that** the reaction is carried out with a mean ratio of water/quaternizing agent throughput of 0.1-1.2, in particular of 0.3-0.8.

8. Process according to one of Claims 1 to 7, **characterized in that** it results in an aqueous solution having a concentration of quaternary salts (I) of 50 to 85% by weight.

9. Process according to one of Claims 1 to 8, **characterized in that** it is carried out in the presence of at least one stabilizer chosen in particular from 3,5-di(tert-butyl)-4-hydroxytoluene, hydroquinone methyl ether, hydroquinone, catechol, tert-butylcatechol, phenothiazine and the mixtures of these stabilizers, the content of stabilizing agent (s) being in particular from 20 to 2 000 ppm, preferably from 100 to 1 200 ppm, with respect to the aqueous solution of quaternary salt (I).

10. Process according to Claim 9, **characterized in that** it is carried out in the presence in addition of at least one sequestering agent for metals chosen in particular from diethylenetriaminepentaacetic acid, the pentasodium salt of diethylenetriaminepentaacetic acid, N-(hydroxyethyl)ethylenediaminetriacetic acid and the trisodium salt of N-(hydroxyethyl)-ethylenediaminetriacetic acid, the content of sequestering agent(s) being in particular from 1 to 100 ppm, preferably from 5 to 30 ppm, with respect to the aqueous solution of quaternary salt (I).

11. Process according to one of Claims 1 to 10, **characterized in that** the residual quaternizing agent is removed by stripping with air.